# EUROPEAN PATENT SPECIFICATION

(11) **EP 3 552 549 B1**
(45) Date of publication and mention of the grant of the patent: **28.10.2020**
(21) Application number: 19167785.5
(22) Date of filing: 08.04.2019
(51) Int. Cl.: A61B 6/00

(54) **X-RAY FLUOROSCOPIC RADIOGRAPHY APPARATUS**
VORRICHTUNG ZUR FLUOROSKOPISCHEN RÖNTGENRADIOGRAFIE
APPAREIL DE RADIOGRAPHIE FLUOROSCOPIQUE À RAYONS X

(30) Priority: 11.04.2018 JP 2018075869
(43) Date of publication of application: 16.10.2019
(73) Proprietor: Shimadzu Corporation, Kyoto-shi, Kyoto 604-8511 (JP)
(72) Inventor: SAKAMOTO, Yuki, Kyoto-shi, Kyoto 6048511 (JP); OKAMOTO, Takeshi, Kyoto-shi, Kyoto 6048511 (JP)
(74) Representative: Mewburn Ellis LLP

(56) References cited:
- EP-A1- 3 206 583
- JP-A- H07 201 491
- US-A1- 2009 154 648

## Description

### TECHNICAL FIELD

The present invention relates to an X-ray fluoroscopic radiography apparatus in which an X-ray is applied to a test object to obtain a fluoroscopic image of the test object in real time.

### BACKGROUND ART

A mobile X-ray fluoroscopic radiography system to be used in examination and operation support has been customarily used in an operating room and other environments (see Patent Literature 1, for example). This system includes a fluoroscopic radiography apparatus that performs fluoroscopy or radiography using an X-ray, and a monitor device that is connected to the fluoroscopic radiography apparatus and displays an X-ray image obtained by the fluoroscopic radiography apparatus. Each of the fluoroscopic radiography apparatus and the monitor device has wheels at its bottom, and is carried to an operating room, examination room, or other locations, depending on the necessity for its operation.

The fluoroscopic radiography apparatus includes a housing provided with wheels at its bottom, and a semicircular shaped arm (C arm) mounted on the top of the housing. The C arm has one end at which an X-ray emitter including an X-ray tube, a collimator, and others is attached, and the other end to which an X-ray detector including, for example, an X-ray image intensifier device, a CCD imaging element, and others is attached. The X-ray emitter and the X-ray detector are disposed to face each other, and a test object is placed between them. Then, the fluoroscopy or radiography is performed. The fluoroscopic radiography apparatus and the monitor device are connected each other through a wire or wireless connection. Image data generated in the fluoroscopic radiography apparatus along with application of the X-ray to the test object is sequentially sent to the monitor device, so as to be displayed, in real time, on a display device, such as a liquid crystal display, provided in the monitor device.

An operation panel for a user (a person in charge of applying the X-ray to a test object) to input and check various settings for the X-ray fluoroscopic radiography is provided in the housing of the fluoroscopic radiography apparatus. The operation panel includes, for example, a touch panel and various operation buttons arranged around the touch panel. The user operates the touch panel or the operation button while checking various bits of information displayed on the touch panel during the fluoroscopy or radiography, whereby the conditions for the X-ray exposure and the conditions for image processing can be changed.

In such an X-ray fluoroscopic radiography system, if tube current (the current flowing through the X-ray tube) is increased, a bright image is obtained, but the exposure dose of a test object is increased. If the tube current is decreased, the exposure dose of the test object is decreased but the image is darkened. Accordingly, when the aforementioned X-ray fluoroscopic radiography system is used for supporting surgery, for example, it is preferable that the user appropriately operates, according to the process of the operation, the operation panel to adjust the tube current, so as to reduce the exposure dose of the test object without disturbing the operation at that time. Document US 2009/154648 A1 discloses a pulsed X-ray fluoroscopic apparatus according to the preamble of claim 1.

### CITATION LIST

### PATENT LITERATURE

Patent Literature 1: JP 2014-131547 A

### SUMMARY OF INVENTION

### TECHNICAL PROBLEM

In the X-ray fluoroscopic radiography system in recent years, it is possible to perform pulsed fluoroscopy in which the X-ray is applied to a test object in a pulsed form for reducing the exposure dose of the test object. In such an X-ray fluoroscopic radiography system, either one of the peak value and the average value of the tube current is displayed, as the condition during the pulsed fluoroscopy, on an operation panel of a fluoroscopic radiography apparatus. However, if an apparatus that displays the peak value of the tube current is used, it is difficult for a user to instantly grasp the average exposure dose of the test object during the fluoroscopy. On the other hand, if an apparatus that displays the average value of the tube current is used, the displayed value of the tube current is changed along with the change in the pulse rate of the X-ray exposure, and thus it is difficult for a user to grasp the amount of the X-ray exposure at every pulse, and to determine an appropriate set value.

The present invention has been made in view of the previously-mentioned problems. The purpose of the present invention is to provide an X-ray fluoroscopic radiography apparatus that allows a user to easily grasp both the average exposure dose of the test object and the amount of the X-ray exposure at every pulse, during the pulsed fluoroscopy.

### SOLUTION TO PROBLEM

The present invention developed for solving the previously described problem is an X-ray fluoroscopic radiography apparatus including:
an X-ray emitter having an X-ray tube, configured to emit an X-ray in a pulsed form;
an X-ray detector configured to detect the X-ray that is emitted from the X-ray emitter and passes through a test object;
an exposure controller configured to control the X-ray emitter;
a display unit; and
a display controller configured to control the display unit to display a peak value of a tube current flowing through the X-ray tube and an average value of the tube current in a predetermined time period while the X-ray is emitted in the pulsed form.

It is preferable for the X-ray fluoroscopic radiography apparatus according to the present invention to include the display controller that controls the display unit to display both the peak value and the average value horizontally or vertically.

In the X-ray fluoroscopic radiography apparatus according to the present invention, the display controller may control the display unit to alternately display the peak value and the average value at a predetermined interval.

The X-ray fluoroscopic radiography apparatus according to the present invention may further include a display switch operation unit. The display controller may control the display unit to change the display states between a state where the peak value is displayed and a state where the average value is displayed, in accordance with operation by a user on the display switch operation unit.

It is preferable for the X-ray fluoroscopic radiography apparatus according to the present invention to include the display unit that is a touch panel.

It is preferable for the X-ray fluoroscopic radiography apparatus according to the present invention to further include:
a support member configured to support the X-ray emitter and the X-ray detector;
a housing to which the support member is attached, the housing containing the exposure controller and the display controller; and
an operation panel provided in the housing, in which the display unit is included in the operation panel.

### ADVANTAGEOUS EFFECTS OF INVENTION

According to an X-ray fluoroscopic radiography apparatus of the present invention, which includes the previously-mentioned configuration, the peak value of a tube current and the average value of the tube current in a predetermined time period are displayed when pulsed fluoroscopy is performed. Accordingly, a user observes these values displayed on a display unit, and can easily grasp the amount of an X-ray exposure at every pulse and the average exposure dose of a test object during the pulsed fluoroscopy.

### BRIEF DESCRIPTION OF DRAWINGS

Fig. 1 is a perspective view of the entire configuration of an X-ray fluoroscopic radiography system according to an embodiment of the present invention.
Fig. 2 is a block diagram showing the main configuration of a fluoroscopic radiography apparatus according to the embodiment.
Fig. 3 is a diagram showing an example of an operation panel provided in the fluoroscopic radiography apparatus, in a state where a peak value of the tube current is shown in an operation screen.
Fig. 4 is a diagram showing a state where an average value of the tube current is displayed on the operation screen.
Fig. 5 is a diagram showing another example of the operation panel provided in the fluoroscopic radiography apparatus.

### DESCRIPTION OF EMBODIMENTS

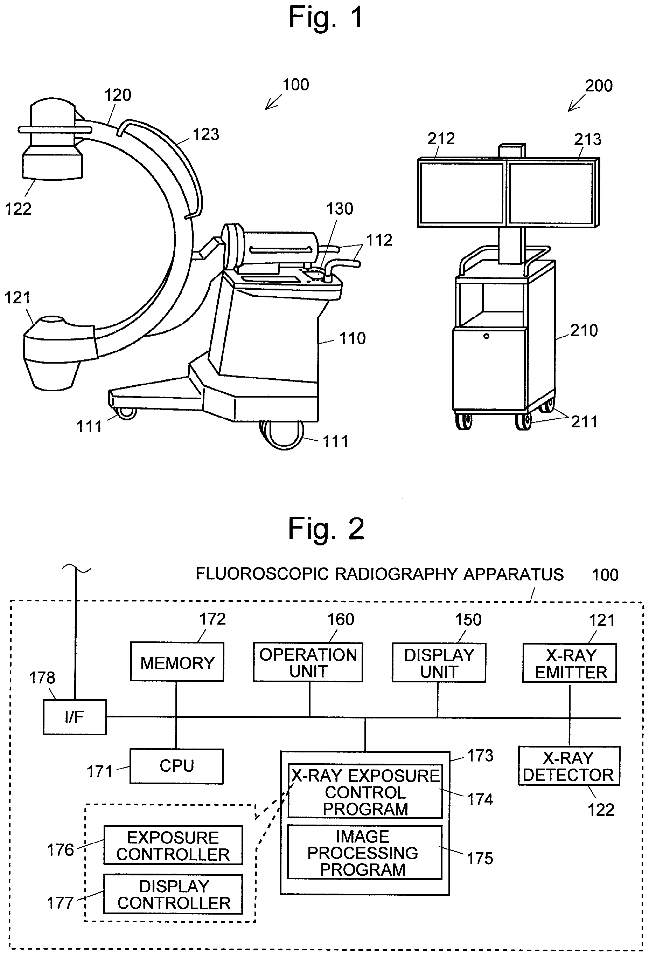

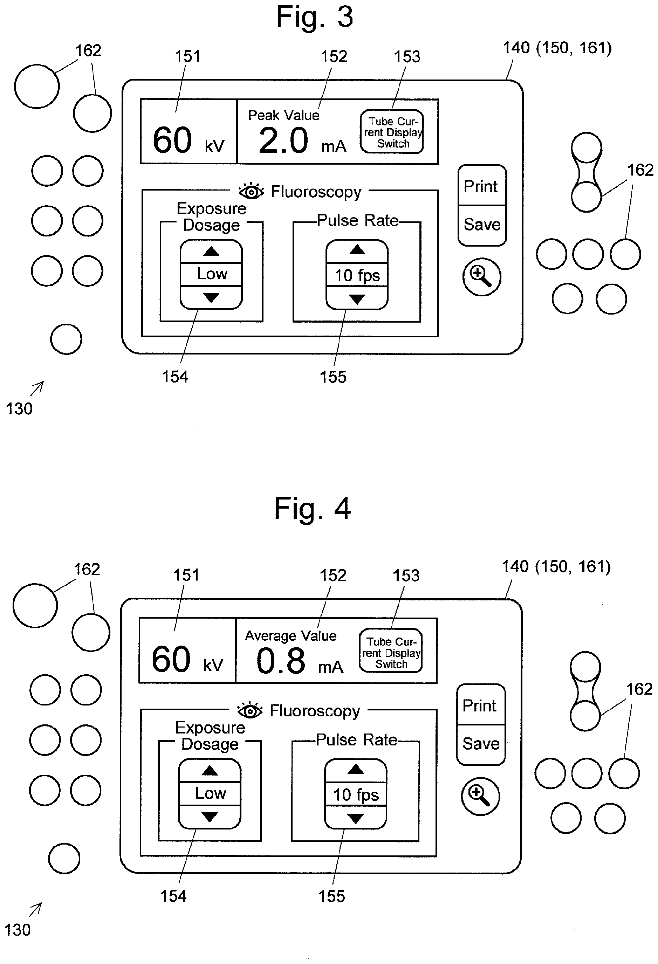

Hereinafter, an embodiment of the present invention is described as follows, with reference to the drawings. Fig. 1 is a perspective view of the configuration of an X-ray fluoroscopic radiography system according to the embodiment of the present invention (corresponding to an X-ray fluoroscopic radiography apparatus in the present invention). Fig. 2 is a functional block view showing a fluoroscopic radiography apparatus included in the system. Figs. 3 and 4 are a plan view showing an operation panel of the fluoroscopic radiography apparatus. The X-ray fluoroscopic radiography system according to the present embodiment includes a fluoroscopic radiography apparatus 100 and a monitor device 200.

The fluoroscopic radiography apparatus 100 includes a housing 110 provided with wheels 111 at its bottom, and a C arm 120 (corresponding to a support member in the present invention) mounted on the top of the housing 110. The C arm has one end at which an X-ray emitter 121 including an X-ray tube, a collimator, and others is attached, and the other end to which an X-ray detector 122 including an X-ray image intensifier device, a CCD imaging element, and others is attached. The housing 110 is provided with, on its top, a traveling handle 112. A user holds the traveling handle 112, and causes the housing 110 to travel, thereby moving the fluoroscopic radiography apparatus 100 to a desired location. An operation handle 123 is fixed on the C arm 120. A user holds the operation handle 123 to cause the C arm 120 to rotate or to move vertically, thereby adjusting the position relationship between a test object (not shown) and the X-ray emitter 121 as well as the X-ray detector 122.

The housing 110 of the fluoroscopic radiography apparatus 100 is provided with, on its top surface, an operation panel 130 for a user, who operates the application of X-ray to the test object, to input or monitor X-ray exposure conditions and image processing conditions. The operation panel 130 includes: a display unit 150 including a liquid crystal panel and others; and an operation unit 160 including a touch sensor 161 attached on the screen of the display unit 150, and operation buttons 162 provided around the display unit 150 (details to be described later).

The housing 110 contains a computer provided with a CPU 171, a memory 172, and a storage device 173 including a mass storage medium, such as a hard disk and a flash memory. The computer is connected to: the X-ray emitter 121 and the X-ray detector 122 which are provided in the aforementioned C arm 120; and the display unit 150 and the operation unit 160 which are provided on the operation panel 130. In the storage device 173, there are stored an X-ray exposure control program 174 for controlling the X-ray emitter 121 and an image processing program 175 for applying predetermined processing to a fluoroscopic image generated based on signals detected in the X-ray detector 122. Fig. 2 shows an exposure controller 176 and a display controller 177, which relate to the X-ray exposure control program 174. These controllers basically work as functional means embodied in the software, in such a manner that the CPU 171 reads the X-ray exposure control program in the memory 172 to cause the program to be executed. The X-ray exposure control program 174 and the image processing program 175 need not be separate programs. These programs may be, for example, a single program, and may be in any form. The computer further includes an interface (I/F) 178 that controls the connection with external devices, and is connected to the monitor device 200 with the interface 178 through a communication cable.

The monitor device 200 includes a housing 210 provided with wheels 211 at its bottom, and a main monitor 212 and a reference monitor 213 each of which includes a liquid crystal display and other parts, and is mounted on the top of the housing 210. The housing 210 contains a computer (not shown) that controls the communication with the fluoroscopic radiography apparatus 100 and the display of images on the main monitor 212 and the reference monitor 213. The computer is connected to the fluoroscopic radiography apparatus 100 through an interface and a communication cable (none of them is shown).

When X-ray fluoroscopy is performed using the X-ray fluoroscopic radiography system according to the present embodiment, the fluoroscopic radiography apparatus 100 is disposed next to a bed (not shown) that is to be used in operation or examination, and then a user adjusts the position and angle of the C arm 120 with his/her hand so that a target part of the test object (test subject) who lies on the bed is positioned between the X-ray emitter 121 and the X-ray detector 122. Thereafter, the user operates the operation panel 130 of the fluoroscopic radiography apparatus 100 to set the exposure conditions of the X-ray, and issues a command, from the operation panel 130, to start the exposure of the X-ray. With this operation, the X-ray is emitted from the X-ray emitter 121 under the control of the exposure controller 176, passes through the test object, and is detected by the X-ray detector 122. In the X-ray detector 122, the X-ray is converted to visible light by the image intensifier device, and the visible light is captured by a CCD camera to obtain a fluoroscopic image of the test object. The data of the fluoroscopic image is sent to the monitor device 200 through the interface 178, and is displayed on the main monitor 212 of the monitor device 200. On the reference monitor 213 of the monitor device 200, a reference image that has been previously prepared is displayed.

Before or during the X-ray fluoroscopy by the X-ray fluoroscopic radiography system according to the present embodiment, the exposure condition of the X-ray can be set or changed by operating the operation panel 130, by a user, which is provided in the housing 110 of the fluoroscopic radiography apparatus 100. The operation panel 130 includes, as shown in Fig. 3, a touch panel 140 and a plurality of operation buttons 162 arranged around the touch panel 140. The touch panel 140 includes a display unit 150 including a liquid crystal panel or others, and a touch sensor 161. The touch sensor 161 is a transparent sheet having a size substantially the same as that of the screen of the display unit 150, and is attached on the screen of the display unit 150.

An operation screen previously determined is displayed on the display unit 150, under the control of the display controller 177. Fig. 3 shows the operation screen during the pulsed fluoroscopy. The operation screen includes a tube voltage display area 151 in which a value of the tube voltage is displayed, a tube current display area 152 in which a value of the tube current is displayed, a exposure dosage selector 154 that is a graphical user interface (GUI) button for a user to make a selection in the exposure dosages of the X-ray, a pulse rate selector 155 that is a GUI button for a user to make a selection in the pulse rates, and so on. Next to the tube current display area 152, there is provided a tube current display switch button 153 that is a GUI button for switching the display on the tube current display area 152 between the display of the peak value of the tube current and the display of the average value of the tube current in a predetermined time period (a time period that has been previously determined). In the operation screen shown in Fig. 3, the peak value of the tube current is displayed on the tube current display area 152. If a user touches the tube current display switch button 153 in this state, the average value of the tube current is displayed on the tube current display area 152, as shown in Fig. 4. If the user touches the tube current display switch button 153 in the state shown in Fig. 4, the peak value of the tube current is displayed on the tube current display area 152, as shown in Fig. 3. It should be noted that the tube current display switch button 153 may be the GUI button as mentioned above, or may be a physical button (mechanical switch) provided in the vicinity of the touch panel 140.

According to the present embodiment as mentioned above, a user pushes down the tube current display switch button 153 provided in the operation panel 130 of the fluoroscopic radiography apparatus 100 before or during the pulsed fluoroscopy, to thereby change the operation screen of the touch panel 140 between the state where the tube current is displayed with its peak value, and the state where the tube current is displayed with its average value. With this configuration, a user in charge of the application of the X-ray to the test object can easily grasp both the amount of the X-ray exposure at every pulse and the average exposure dose of the test object during the pulsed fluoroscopy, based on these values displayed on the operation panel 130 in his/her hand.

Although the embodiment for carrying out the present invention is described above with specific examples, the present invention is not limited to the above-mentioned examples. For example, though the operation by a user on the tube current display switch button 153 switches the states of the display on the touch panel 140 between the state where the peak value of the tube current is displayed and the state where the average value of the tube current is displayed in the foregoing embodiment, both states may be switched in accordance with the predetermined operation by a user other than the pressing down of the tube current display switch button 153. As a specific example, the peak value of the tube current may be displayed on the tube current display area 152 while a user operates the pulse rate selector 155, and the average value of the tube current may be displayed during the situation other than the state where the user operates the pulse rate selector 155.

In addition, both states may be automatically changed at fixed intervals, in place of the change in display on the tube current display area 152, in accordance with the operation by a user, between the state where the peak value of the tube current is displayed and the state where the average value is displayed.

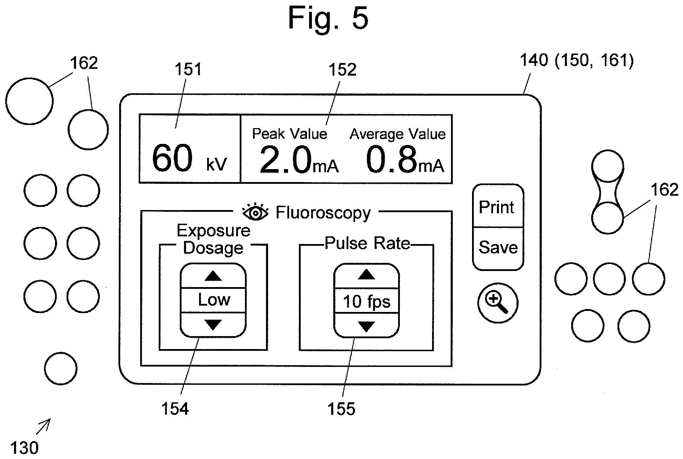

Furthermore, the peak value and the average value of the tube current may be both displayed on the tube current display area 152 provided in the touch panel 140, as shown in Fig. 5. Although Fig. 5 shows a state where the peak value and the average value are arranged horizontally, they may be arranged vertically.

In the embodiments mentioned above, the peak value and the average value of the tube current are displayed on the display unit 150 of the operation panel 130 provided in the fluoroscopic radiography apparatus 100. However, the present invention is not limited thereto. For example, the peak value and the average value of the tube current may be displayed on the main monitor 212 or the reference monitor 213 of the monitor device 200. Alternatively, these values may be displayed on a display unit provided in the monitor device 200 in addition to the main monitor 212 and the reference monitor 213.

### REFERENCE SIGNS LIST

- 100...: Fluoroscopic Radiography Apparatus
110... Housing
120... C Arm
121... X-Ray Emitter
122... X-Ray Detector
130... Operation Panel
140... Touch Panel
150... Display Unit
151... Tube Voltage Display Area
152... Tube Current Display Area
153... Tube Current Display Switch Button
154... Exposure Dosage Selector
155... Pulse Rate Selector
160... Operation Unit
161... Touch Sensor
162... Operation Button
- 171...: CPU
- 172...: Memory
- 173...: Storage Device
174... X-Ray Exposure Control Program
175... Image Processing Program
176... Exposure Controller
177... Display Controller
178... Interface

- 200...: Monitor Device
212... Main Monitor
213... Reference Monitor

## Claims

1. An X-ray fluoroscopic radiography apparatus, comprising:
an X-ray emitter having an X-ray tube, configured to emit an X-ray in a pulsed form;
an X-ray detector configured to detect the X-ray that is emitted from the X-ray emitter and passes through a test object;
an exposure controller configured to control the X-ray emitter;
a display unit; **characterised in that** it further comprises
a display controller configured to control the display unit to display a peak value of a tube current flowing through the X-ray tube and an average value of the tube current in a predetermined time period while the X-ray is emitted in the pulsed form.

2. The X-ray fluoroscopic radiography apparatus according to claim 1, wherein
the display controller controls the display unit to display both the peak value and the average value horizontally or vertically.

3. The X-ray fluoroscopic radiography apparatus according to claim 1, wherein
the display controller controls the display unit to alternately display the peak value and the average value at a predetermined interval.

4. The X-ray fluoroscopic radiography apparatus according to claim 1, further comprising a display switch operation unit, wherein
the display controller controls the display unit to change display states between a state where the peak value is displayed and a state where the average value is displayed, in accordance with operation by a user on the display switch operation unit.

5. The X-ray fluoroscopic radiography apparatus according to any one of claims 1 to 4, wherein
the display unit is a touch panel.

6. The X-ray fluoroscopic radiography apparatus according to any one of claims 1 to 5, further comprising:
a support member configured to support the X-ray emitter and the X-ray detector;
a housing to which the support member is attached, the housing containing the exposure controller and the display controller; and
an operation panel provided in the housing, wherein
the display unit is included in the operation panel.

## Patentansprüche

1. Vorrichtung für fluoroskopische Röntgenradiografie, die Folgendes umfasst:
einen Röntgenstrahlenstrahler, der eine Röntgenstrahlenröhre aufweist, der ausgelegt ist, um einen Röntgenstrahl in gepulster Form auszustrahlen;
einen Röntgenstrahlendetektor, der ausgelegt ist, um die Röntgenstrahlung, die von dem Röntgenstrahlenstrahler ausgestrahlt wird und durch ein Testobjekt hindurchgeht, zu detektieren;
eine Bestrahlungssteuerung, die ausgelegt ist, um den Röntgenstrahlenstrahler zu steuern;
eine Anzeigeeinheit;
**dadurch gekennzeichnet, dass** sie ferner Folgendes umfasst:
eine Anzeigesteuerung, die ausgelegt ist, um die Anzeigeeinheit zu steuern, um einen Peakwert eines Röhrenstroms, der durch die Röntgenstrahlröhre fließt, und einen Mittelwert des Röhrenstroms in einer vorbestimmten Zeitspanne anzuzeigen, während die Röntgenstrahlung in gepulster Form ausgestrahlt wird.

2. Vorrichtung für fluoroskopische Röntgenradiografie nach Anspruch 1, wobei die Anzeigesteuerung die Anzeigeeinheit steuert, um den Peakwert sowie den Mittelwert horizontal oder vertikal anzuzeigen.

3. Vorrichtung für fluoroskopische Röntgenradiografie nach Anspruch 1, wobei die Anzeigesteuerung die Anzeigeeinheit steuert, um den Peakwert und den Mittelwert in einem vorbestimmten Zeitintervall abwechselnd anzuzeigen.

4. Vorrichtung für fluoroskopische Röntgenradiografie nach Anspruch 1, die ferner eine Anzeigeschaltbetriebseinheit umfasst, wobei die Anzeigesteuerung die Anzeigeeinheit steuert, um Anzeigezustände auf der Anzeigeschaltbetriebseinheit zwischen einem Zustand, in dem der Peakwert angezeigt wird, und einem Zustand, in dem der Mittelwert angezeigt wird, gemäß der Bedienung durch einen Benutzer zu ändern.

5. Vorrichtung für fluoroskopische Röntgenradiografie nach einem der Ansprüche 1 bis 4, wobei die Anzeigeeinheit ein Berührungsbildschirm ist.

6. Vorrichtung für fluoroskopische Röntgenradiografie nach einem der Ansprüche 1 bis 5, die ferner Folgendes umfasst:
ein Stützelement, das ausgelegt ist, um den Röntgenstrahlenstrahler und den Röntgenstrahlendetektor abzustützen;
ein Gehäuse, an dem das Stützelement angebracht ist, wobei das Gehäuse die Bestrahlungssteuerung und die Anzeigesteuerung enthält; und
ein Bedienfeld, das in dem Gehäuse bereitgestellt ist, wobei
die Anzeigeeinheit in dem Bedienfeld integriert ist.

## Revendications

1. Appareil de radiographie fluoroscopique à rayons X, comprenant :
un émetteur de rayons X disposant d'un tube à rayons X, configuré pour émettre des rayons X sous une forme puisée ;
un détecteur de rayons X configuré pour détecter des rayons X qui sont émis par l'émetteur de rayons X et passent à travers un objet de test ;
un dispositif de commande d'exposition configuré pour commander l'émetteur de rayons X ;
une unité d'affichage ;
**caractérisé en ce qu'**il comprend en outre
un dispositif de commande d'affichage configuré pour commander l'unité d'affichage afin d'afficher une valeur de crête d'un courant de tube circulant à travers le tube à rayons X et une valeur moyenne du courant de tube pendant une durée prédéterminée pendant que les rayons X sont émis sous la forme pulsée.

2. Appareil de radiographie fluoroscopique à rayons X selon la revendication 1, dans lequel
le dispositif de commande d'affichage commande l'unité d'affichage pour afficher à la fois la valeur de crête et la valeur moyenne horizontalement ou verticalement.

3. Appareil de radiographie fluoroscopique à rayons X selon la revendication 1, dans lequel
le dispositif de commande d'affichage commande l'unité d'affichage pour afficher en alternance la valeur de crête et la valeur moyenne à un intervalle prédéterminé.

4. Appareil de radiographie fluoroscopique à rayons X selon la revendication 1, comprenant en outre une unité d'actionnement de commutation d'affichage, dans lequel
le dispositif de commande d'affichage commande l'unité d'affichage pour changer des états d'affichage entre un état dans lequel la valeur de crête est affichée et un état dans lequel la valeur moyenne est affichée, conformément à un actionnement par un utilisateur sur l'unité d'actionnement de commutation d'affichage.

5. Appareil de radiographie fluoroscopique à rayons X selon l'une quelconque des revendications 1 à 4, dans lequel
l'unité d'affichage est un panneau tactile.

6. Appareil de radiographie fluoroscopique à rayons X selon l'une quelconque des revendications 1 à 5, comprenant en outre :
un élément de support configuré pour supporter l'émetteur de rayons X et le détecteur de rayons X ;
un boîtier auquel l'élément de support est fixé, le boîtier contenant le dispositif de commande d'exposition et le dispositif de commande d'affichage ; et
un panneau de commande prévu dans le boîtier, dans lequel
l'unité d'affichage est incluse dans le panneau de commande.
